Europäisches Patentamt

(19) European Patent Office

Office européen des brevets

(11) Numéro de publication: **0 185 027**
**B1**

(12) **FASCICULE DE BREVET EUROPEEN**

(45) Date de publication du fascicule du brevet: **26.09.90**

(21) Numéro de dépôt: **85901962.2**

(22) Date de dépôt: **14.05.85**

(86) Numéro de dépôt international:
**PCT/CH85/00080**

(87) Numéro de publication internationale:
**WO 85/05269 05.12.85 Gazette 85/26**

(51) Int. Cl.⁵: **A 61 J 1/06,** B 65 B 31/02

(54) PROCEDE DE FABRICATION D'UNE SERINGUE PREREMPLIE A DOSE UNITAIRE ET DISPOSITIF POUR LA MISE EN O EUVRE DE CE PROCEDE.

(30) Priorité: **16.05.84 CH 2399/84**

(43) Date de publication de la demande:
**25.06.86 Bulletin 86/26**

(45) Mention de la délivrance du brevet:
**26.09.90 Bulletin 90/39**

(84) Etats contractants désignés:
**AT BE CH DE FR GB IT LI LU NL SE**

(56) Documents cités:
**FR-A- 136 505**
**FR-A-1 431 184**
**US-A-2 823 500**
**US-A-2 971 509**
**US-A-3 005 300**
**US-A-3 378 008**
**US-A-3 538 672**

(73) Titulaire: **COSMONOR S.A.**
**10B, chemin des Princes**
**CH-1222 Vésenaz (CH)**

(72) Inventeur: **MEYER, Gabriel**
**10A, chemin des Princes**
**CH-1222 Vésenaz (CH)**
Inventeur: **HOWALD, Ernst**
**10B, chemin des Princes**
**CH-1222 Vésenaz (CH)**

(74) Mandataire: **Nithardt, Roland**
**CABINET ROLAND NITHARDT Attn: Cabinet**
**MOSER & CIE Rue Edouard Verdan 15**
**CH-1400 Yverdon-les-Bains (CH)**

## Description

La présente invention concerne un procédé de fabrication d'une seringue préremplie à dose unitaire, comprenant d'une part une carpule ouverte à une de ses extrémités, pourvue d'un col de section rétrécie, destinée à contenir un médicament à injecter, et d'autre part un injecteur composé d'une capsule adaptable sur le col de la carpule et d'un piston-vanne solidaire de la capsule et destiné à être inséré à l'intérieur de la caruple, procédé dans lequel on amène par deux voies différentes, dans une même station d'assemblage, la carpule contenant le médicament à injecter et l'injecteur, on positionne ces deux composants l'un au-dessus de l'autre et on les approche pour les assembler, et dans lequel on procède, antérieurement à l'assemblage des composants, dans ladite station d'assemblage, à un gazage de la carpule ou de la carpule et de l'injecteur.

Elle concerne également un dispositif de fabrication d'une seringue préremplie à dose unitaire, pour la mise en oeuvre de ce procédé, comportant des moyens pour gazer la carpule ou la carpule et l'injecteur, avant et pendant l'assemblage de ces deux composants.

On connaît de nombreuses installations automatiques d'assembage de seringues à usage unique généralement constituées par un corps de seringue et par un piston engagé dans ce corps. Ces installations sont généralement de conception classique, les précautions à prendre pour éviter la contamination des composants ainsi que pour effectuer le contrôle de l'assemblage étant bien connues et les moyens à mettre en ouevre étant relativement simples.

Il en va tout différemment des seringues préremplies du type à dose unitaire dont les composants doivent être manipulés mécaniquement avec des précisions et un soin particuliers et où des opérations nouvelles telles que par exemple le remplissage des carpules interviennent en cours de montage.

Les exigences en matière de remplissage sont extrêmement strictes, étant donné que l'objectif à atteindre consiste à introduire le médicament dans la seringue et à sceller hermétiquement cette dernière de telle manière que l'atmosphère surmontant le médicament dans la seringue soit constituée exclusivement d'un gaz choisi en fonction du médicament, par exemple neutre, c'est-a-dire qu'elle soit exempte de particules solides, de contaminants, d'oxygène et de substances oxygénées.

Pour remplir ces exigences on pourrait bien entendu utiliser les techniques connues consistant à monter l'ensemble de la machine dans une salle à atmosphère neutre contrôlée. En raison du coût des installations et des importants moyens techniques à mettre en oeuvre, le choix d'une telle solution constitue un handicap sérieux à une diffusion très large des seringues préremplies à dose unitaire.

Une autre technique a été utilisée pour effectuer un gazage de la carpule après son remplissage et pendant la mise en place du piston dans certaines seringues préremplies comportant une carpule qui constitue en fait le corps de la seringue et un piston qui obture l'une des extrémités de ce corps. Cette technique consiste à pincer le piston entre deux mâchoires de façon à réduire son diamètre, et à insuffler un gaz choisi entre la paroi intérieure du corps de la seringue et le piston, sur un des côtés de ce dernier, de telle manière que ce gaz puisse s'échapper de l'autre côté.

Cette façon de procéder ne fournit pas les garanties que le gazage ne draine pas des particules solides ou de l'oxygène vers l'intérieur du corps de la seringue et ne permet pas d'engendrer une surpression du gas à l'intérieur de la seringue.

En outre, les techniques actuelles ne permettant pas de contrôler qualitativement ni quantitativement cette opération de gazage des carpules, et en conséquence, d'arrêter la production en cas de défectuosités constatées.

Une autre exigence imposée au procédé est celle du contrôle des produits fabriqués. On sait que les ampoules scellées contenant actuellement la plupart des médicaments destinés à l'injection, sont soumises à un contrôle optique permettant en principe de vérifier l'absence de particules solides, et un contrôle d'étanchéité dit "contrôle du bain bleu" qui consiste à tremper les ampoules dans un bain liquide coloré et de vérifier individuellement chaque ampoule pour détecter l'éventuelle présence de la solution colorée dans l'ampoule de médicament.

Il va sans dire que ce contrôle est fastidieux et coûteux et qu'en outre son efficacité peut, dans certains cas, être mise en doute.

La présente invention se propose de pallier les inconvénients des systèmes connus et de développer un procédé simple et efficace de fabrication de seringues préremplies à dose unitaire, satisfaisant à la fois aux exigences les plus strictes en matière de remplissage ainsi qu'à celles concernant le contrôle en cours de fabrication et le contrôle final des produits.

Dans ce but, le procédé selon l'invention est caractérisé en ce qu'on effectue le gazage en engendrant à l'intérieur d'une cavité contenant entièrement ladite carpule, un courant de gaz circulant de bas en haut en balayant les parois extérieures de la carpule, et en provoquant à travers des canaux de l'injecteur un flux de gaz de balayage, en ce qu'on effectue un contrôle qualitatif et/ou quantitatif du gaz pendant ou après le gazage, et en ce qu'on effectue un contrôle final de la seringue après l'assemblage des dits composants, en exerçant une poussée sur l'un de ces composants en en contrôlant au moins le retour de ce composant vers sa position initiale, la dit retour étant déclenché par la supression du gaz contenue dans la carpule.

Selon une forme de mise en oeuvre préférée de ce procédé, au moins une phase de gazage de l'injecteur comprend le balayage des canaux par un flux ascendant de gaz surmontant initialement le liquide contenu dans la carpule, qui est refoulé vers les canaux lors de la mise en place de l'injecteur.

La courant de gas balayant les parois extérieures de la carpule a de préférence une vitesse supérieure à la vitesse d'un flux laminaire dans lequel se trouve l'installation, et susceptible d'entraîner des particules contaminantes.

Selon un mode de réalisation particulièrement avantageux, un provoque le courant de gaz de balayage en refoulant dans la cavité contenant la carpule, une quantité prédéterminée de gaz choisi stockée dans une chambre de dosage.

Selon un autre mode de réalisation avantageux, on provoque le courant de gaz de balayage en alimentant en continu la cavité contenant la carpule au moyen d'un gaz choisi.

Pour créer une surpression à l'intérieur de la carpule, on peut obturer la cavité contenant la carpule et on engendre ainsi le courant de gaz de balayage à une pression supérieure à la pression atmosphérique.

Pour effectuer le gazage de l'injecteur on engendre, au cours d'au moins une phase de l'opération, un flux descendant de gaz de balayage, à travers les canaux de cet injecteur.

On peut également maintenir l'injecteur dans une chambre contenant du gaz choisi, pendant sa mise en place sur la carpule.

Selon un mode de réalisation avantageux, on effectue le contrôle du gaz sous forme statique par des interventions ponctuelles à fréquence déterminée.

Le contrôle final des seringues est de préférence effectué en exerçant une force déterminée, axialement sur la carpule ou sur l'injecteur préalablement assemblés, et on détecte à la fois la valeur de l'enfoncement relatif de ces deux composants et la valeur du retour de ces composants dans leur position initiale.

Pour compléter la mesure et faciliter l'interprétation des résultats, on détecte également la somme algébrique des deux valeurs de l'enfoncement relatif et du retour des composants dans leur position initiale.

Ladite force déterminée est de préférence exercée par une masselotte de masse prédéterminée.

Selon un mode de réalisation préféré du dispositif selon l'invention, la cavité de gazage a une profondeur supérieure à la hauteur de la carpule, et le dispositif comporte des moyens pour amener un gaz choisi dans cette cavité et des moyens pour engendrer un courant de balayage de ce gaz, circulant de bas en haut entre les parois de la cavité et celles de la carpule.

La cavité de gazage comporte avantageusement une chambre de dosage ménagée sous la cavité de gazage, cette chambre de dosage étant alimentée en gaz choisi et contenant initialement un volume prédéterminé de ce gaz à une pression donnée.

Pour permettre le transport des carpules la cavité de gazage est ménagée dans une unité centrale de support et de transfert, et cette unité centrale se présente sous la forme d'une table circulaire rotative ou rectiligne à déplacement linéaire.

L'unité centrale de support et de transfert comporte de préférence plusieurs cavités, chacune de ces cavités étant constituée par une entaille ménagée à la périphérie de ladite unité et comportant des moyens pour fermer sélectivement cette entaille pour réaliser une cavité cylindrique.

Selon un mode de réalisation particulier, l'unité centrale de support et de transfert se décompose en deux blocs adjacents dont l'un est fixe et dont l'autre est mobile en translation, chacun de ces blocs comportant une série d'encoches disposées de manière à pouvoir être amenées en regard les unes des autres afin que les carpules et/ou les injecteurs et/ou les seringues puissent être transférés d'une encoche à l'autre et déplacés linéairement avec le bloc mobile en translation.

Selon un autre mode de réalisation, l'unité centrale de support et de transfert est associée à une seconde unité de transport, et comporte une série d'encoches périphériques comportant chacune une porte rotative agencée pour coopérer avec l'encoche correspondante pour former ladite cavité de gazage.

Les moyens de contrôle final comportent de préférence deux capteurs dimensionnels montés l'un sur un bâti fixe, l'autre sur une coulisse mobile axialement par rapport à ce bâti fixe.

Ces moyens de contrôle final comportent avantageusement trois indicateurs agencés pour indiquer respectivement l'enfoncement relatif de la carpule et de l'injecteur, le retour de l'injecteur par rapport à la carpule et la somme algébrique des deux valeurs précédentes.

Les moyens pour effectuer un contrôle quantitatif du gaz contenu dans la carpule comportent de préférence un capteur volumétrique et des moyens pour mettre en communication l'intérieur de la carpule avec ce capteur.

Les moyens pour effectuer un contrôle qualitatif du gaz contenu dans la carpule comportent de préférence un compteur de particules et/ou un détecteur à pression partielle de gaz.

La cavité de gazage est avantageusement raccordée à un détecteur à pression partielle de gaz et/ou à un compteur de particules.

La présente invention sera mieux comprise en référence à la description d'exemples de réalisation et du dessin annexé dans lequel:

La figure 1A représente une vue en coupe axiale d'une première forme de réalisation d'une seringue préremplie à dose unitaire que le procédé et le dispositif selon l'invention se proposent d'assembler,

La figure 1B représente une vue en coupe axiale d'une seconde forme de réalisation d'une seringue préremplie à dose unitaire que le procédé et le dispositif selon l'invention se proposent d'assembler,

La figure 2 représente une vue schématique d'une première forme de réalisation du dispositif selon l'invention,

La figure 3 représente une vue schématique d'une autre forme de réalisation du dispositif selon l'invention,

Les figures 4A, 4B et 4C illustrent les trois phases principales de la mise en place de l'injecteur sur la carpule,

La figure 5 est une vue en coupe partielle d'une forme de réalisation de l'unité centrale de support et de transfert illustrant plus particulièrement la cavité réceptrice des carpules,

La figure 6 représente une vue en coupe d'une autre forme de réalisation des cavités réceptrices de carpules,

La figure 7 représente une variante du mécanisme d'enclipsage associé aux cavités réceptrices de carpules,

La figure 8 représente une vue en coupe transversale d'une forme de réalisation particulière de l'unité centre de support et de transfert des carpules,

La figure 9 représente une variante de cette unité centrale de support et de transfert,

La figure 10 représente une autre forme de réalisation de cette unité centrale de support et de transfert des carpules, illustrant plus particulièrement un mode d'amenée du gaz choisi dans la cavité contenant les carpules,

La figure 11 illustre une forme de réalisation du dispositif selon l'invention, et plus particulièrement une variante dans laquelle est prévue une cloche de protection recouvrant l'injecteur,

La figure 12 illustre un mode de réalisation dans lequel l'unité centrale de support et de transfert des composants est constituée par une table rotative,

La figure 13 représente une autre forme de réalisation dans laquelle cette unité est constitée par une table de transfert linéaire,

La figure 13A représente une phase intermédiaire de travail de l'unité centrale de support et de transfert représentée par la fig. 13,

La figure 14 représente un mode de transfert, des carpules d'une première unité centrale vers une seconde unité de transport,

La figure 15 représente une vue schématique d'une forme de réalisation préférée de l'unité de contrôle dynamique effectuant le contrôle final des seringues,

La figure 16 illustre un dispositif permettant d'effectuer un contrôle quantitatif du gazage, et

La figure 17 illustre un dispositif permettant d'effectuer un contrôle qualitatif du gazage.

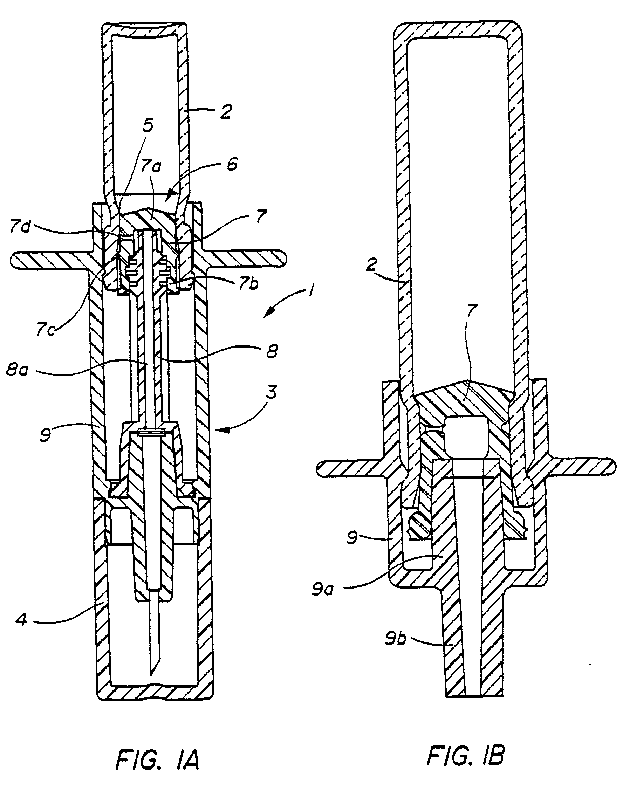

La figure 1A représente une première forme de réalisation d'une seringue 1 préremplie à dose unitaire que l'on se propose de réaliser par le procédé défini ci-dessus. Elle se compose essentiellement d'une carpule 2, d'un injecteur 3 et d'un cache-embout porte-aiguille 4. La carpule 2 comporte un col 5 de section rétrécie. L'injecteur comprend un piston-vanne 6 composé essentiellement d'un organe d'obturation souple 7 monté sur un organe de raidissement rigide 8 et une capsule 9 solidaire du piston-vanne 6 et adaptable par-dessus le col de la carpule. L'organe d'obturation souple 7 comprend une tête de piston 7a solidaire d'une jupe annulaire 7b pourvue d'au moins un bourrelet d'étanchéité 7c. Un canal radial 7d, ménagé à travers la jupe annulaire communique avec un canal axial 8a ménagé dans l'organe de raidissement rigide 8. Un capuchon 4' protège-carpule peut être adapté sur la carpule et fixé par des moyens amovibles (non représentés) à la capsule 9.

La fig. 1B représente une seconde forme de réalisation d'une seringue préremplie à dose unitaire que l'on se propose de réaliser par le procédé ci-dessus. Elle se compose essentiellement d'une carpule 2 contenant un médicament liquide sous pression d'un gaz choisi. L'organe d'obturation souple 7 est directement monté sur un embout de raidissement court 9a solidaire de la capsule 9 et prolongé par un embout porte-aiguilles 9b.

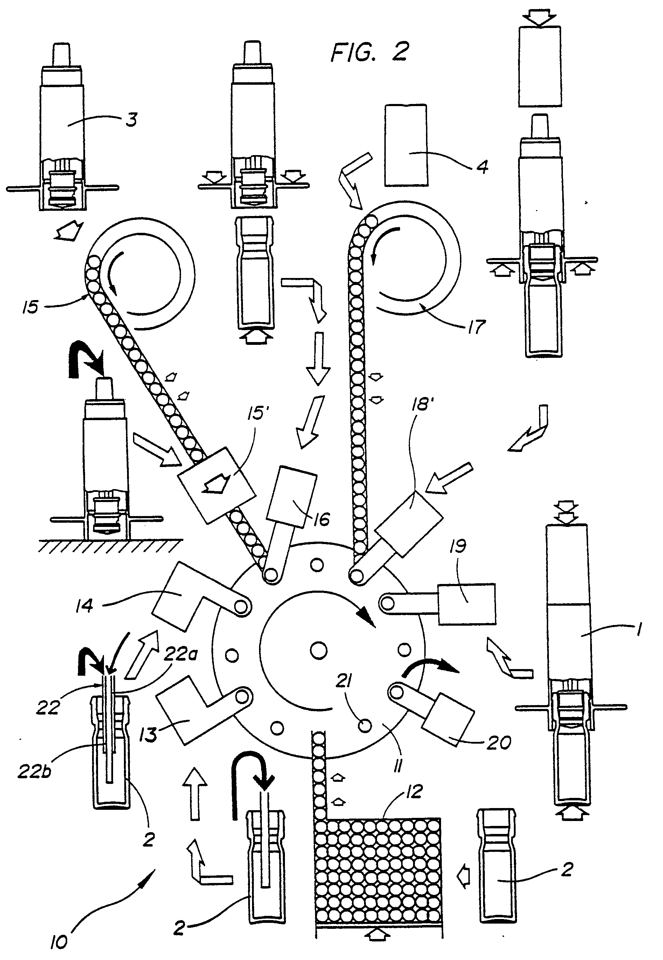

La figure 2 illustre sous forme schématique le dispositif 10 de fabrication d'une seringue préremplie à dose unitaire telle que définie précédemment. Ce dispositif comporte essentiellement une unité centrale 11 de support et de transfert des composants amenés et traités dans une série de stations de travail qui, dans l'exemple illustré par la figure sont les suivantes:

a) une station 12 d'amenée des carpules 2,

b) une station 13 de prégazage des carpules 2,

c) une station 14 de remplissage-gazage des carpules 2,

d) une station 15 d'amenée des injecteurs 3, couplée avec une station 15' de gazage des injecteurs,

e) une station 16 d'assemblage des carpules 2 et des injecteurs 3,

f) une station 17 d'amenée des cache-embouts porte-aiguilles 4,

g) une station 18 de mise en place desdits cache-embouts porte-aiguilles 4,

h) une station 19 de contrôle final de la seringue 1, et

i) une station 20 d'évacuation des seringues acceptées par la station de contrôle final.

Dans cet exemple de réalisation, les carpules 2 sont logées dans des cavités 21 ménagées le long de la périphérie de l'unité centrale de support et de transfert 11. Les carpules sont de ce fait les organes directeurs qui entraînent les injecteurs après le passage dans la station 16. Ce choix permet, comme cela sera décrit plus en détail par la suite, d'effectuer un gazage des carpules pendant toute la phase de mise en place de l'injecteur, et, dans certains cas, le maintien d'une pression de gaz supérieure à la pression atmosphérique au-dessus du médicament liquide. Le prégazage dans la station 13 a pour but de "laver" les carpules au moyen d'un gaz choisi tel que par exemple le $CO_2$, le plus couramment utilisé. Le remplissage-gazage effectué dans la station 14 s'effectue de préférence au moyen d'une aiguille double 22 dont le canal central 22a sert au remplissage de la carpule et dont le canal annulaire périphérique 22b assure l'amenée de gaz en vue du gazage de la carpule.

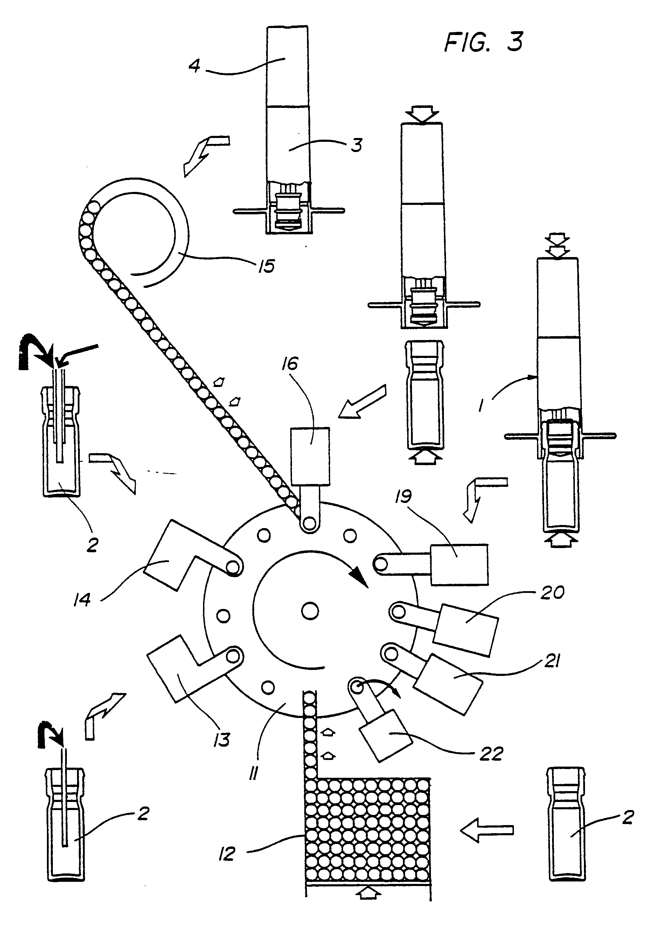

Une variante de ce dispositif est illustrée par la fig. 3. Ce dispositif comporte comme précédemment une unité centrale 11 de support et de transfert des composants, ainsi que les stations suivantes:

a) une station 12 d'amenée des carpules 2,

b) une station 13 de prégazage des carpules 2,

c) une station 14 de remplissage-gazage des carpules 2,

d) une station 15 d'amenée des injecteurs 3, équipés de leurs cache-embouts porte-aiguilles 4, (cette station n'est donc, dans ce cas, pas couplée avec une station de gazage des injecteurs),

e) une station 16 d'assemblage des carpules 2 et des injecteurs 3,

f) une station 19 de contrôle final de la seringue 1,

g) une station 20 de contrôle quantitatif du gazage des seringues,

h) une station 21 de contrôle qualitatif du gazage des seringues,

i) une station 22 d'évacuation des seringues acceptées par la station de contrôle final.

Les stations d'amenée 17 et de mise en place 18 des cache-embouts porte-aiguilles 4 ont été supprimées, étant donné que les injecteurs sont amenés par la station 15, déjà équipés desdits cache-embouts porte-aiguilles.

Il est évident que cette variante de réalisation sous la forme représentée ne permet pas le gazage de l'injecteur au cours de sa mise en place sur la carpule.

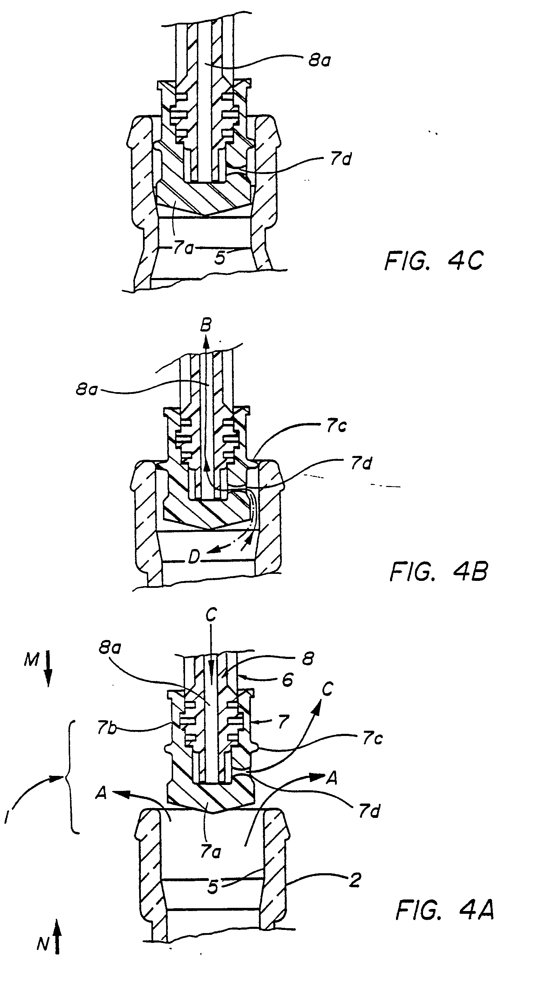

Les fig. 4A, 4B et 4C illustrent les trois phases principales de la mise en place de l'injecteur sur la carpule dans le cas où seule la carpule est gazée et dans le cas où les deux composants, à savoir la carpule et l'injecteur, sont simultanément gazés.

Ces figures montrent la seringue 1 représentée partiellement. La carpule 2 prégazée dans la station 13 est remplie et gazée dans la station 14. Dans la station 12, elle a été préalablement introduite dans une cavité qui est également gazée (voir description détaillée ci-dessous), de telle manière qu'un flux de gaz choisi s'écoule de bas en haut autour de la carpule, en empêchant toute pénétration de particules et d'air et en particulier d'oxygène dans la carpule. De ce fait, on peut admettre que l'espace intérieur de la carpule surmontant le médicament liquide est entièrement rempli de gaz choisi, au moment où l'injecteur est amené au-dessus de l'ouverture de la carpule. Lorsque l'injecteur est descendu dans le sens de la flèche M (ou lorsque la carpule est poussée vers le haut dans le sens de la flèche N), la réduction de volume libre induite par l'insertion de la tête de piston 7a, provoque un échappement de gaz dans le sens des flèches A. En vertu de la loi de Henry relative à l'équilibre des pressions partielles dans les mélanges des gaz, l'oxygène contenue dans l'air ambiant aurait tendance, à l'état statique, à pénétrer dans l'espace surmontant le liquide à l'intérieur de la carpule. Pour éviter une telle migration de l'oxygène en cours et après le remplissage de la carpule, on provoque un flux de gaz choisi de l'intérieur vers l'extérieur. Ce flux engendre un courant de balayage empêchant toute pénétration d'air, et en particulier d'oxygène, ainsi que toute pénétration de particules solides à l'intérieur de la carpule.

Ce courant continue à se produire jusqu'au moment où le bourrelet d'étanchéité 7c entre en contact avec le bord de la carpule (voir fig. 4B). Le premier point d'étanchéité est atteint. Si l'on continue à déplacer la carpule et/ou l'injecteur l'un par rapport à l'autre, le volume susceptible d'être occupé par le gaz surmontant le médicament continue à se réduire. Une partie de ce gaz choisi s'échappe à travers le canal radial 7d et gagne le canal axial 8a, comme le montre la flèche B. Ceci a pour conséquence de "rincer" à l'aide de gaz choisi les canaux d'injection et d'éviter ainsi toute pénétration de particules ou d'oxygène par cette voie.

Si l'on continue à déplacer la carpule et/ou l'injecteur l'un vers l'autre, la tête de piston 7a s'engage dans la zone du col à diamètre rétréci pour atteindre le second point d'étanchéité illustré par la fig. 4C. A partir de ce point, le déplacement relatif de la carpule et de l'injecteur, qui engendre une diminution de volume de gaz surmontant le liquide médicamenteux, se traduit par une augmentation de la pression de ce gaz à l'intérieur de la carpule. En effet, dès que le second point d'étanchéité est atteint, le canal radial est obturé, ce qui empêche toute fuite de gaz par cette voie.

Dans la pratique, on continuera à enfoncer l'injecteur jusqu'à une position dans laquelle la tête du piston 7a est comprimée au maximum dans la zone de section rétrécie du col de la carpule, alors que le bourrelet ou lèvre d'étanchéité 7c reste positionné dans la zone large prolongeant ce col rétréci, afin d'éviter une trop forte compression de ce bourrelet pendant la phase de stockage. Cette forte compression de la tête de piston constitue dans la pratique une excellente protection contre la migration de gaz indésirables tels que par exemple l'oxygène vers l'intérieur de la carpule pendant le stockage de la seringue.

Le cas décrit ci-dessus correspond en fait à la réalisation selon la fig. 3. c'est-à-dire à celle où les injecteurs sont amenés dans la station 15, équipés du cache embout porte-aiguille 4, et où l'on n'effectue pas le gazage de l'injecteur pendant l'enclipsage de cet injecteur sur la carpule, par exemple parce que l'injecteur a été prégazé dans une station antérieure.

Dans le cas où un tel gazage est effectué, on injecte du gaz choisi dans le canal axial 8a. Ce gaz "rince" le canal axial puis le canal radial 7d at s'échappe initialement dans le sens de la flèche C. Lorsqu'on se trouve dans la phase illustrée par la fig. 4B, le gaz choisi injecté à travers le canal axial est contraint de pénétrer à l'intérieur de la carpule dans le sens de la flèche D. Ce moyen permet, entre autres, de créer une surpression réglable qui peut être relativement importante à l'intérieur de cette carpule. Dès que la tête de piston 7a atteint la position illustrée par la fig. 4C, l'injection de gaz choisi à l'intérieur de la carpule par la voie du canal axial 8a et du canal 7d est arrêtée.

Comme mentionné précédemment, il est nécessaire d'engendrer un flux de gaz inerte s'échappant de l'intérieur de la carpule pour éviter une pénétration d'air ou de particules solides dans cette carpule au moment de la mise en place de l'injecteur. Le risque d'une pénétration accidentelle de particules et d'oxygène dans l'atmosphère de gaz choisi surmontant le médicament est encore minimisé si l'on opère dans une enceinte close de gaz choisi. Des conditions similaires peuvent être atteintes lorsqu'on loge entièrement la carpule dans une cavité cylindrique et qu'on balaye l'espace entre les parois intérieures de la cavité et les parois extérieures de la carpule par un flux ascendant de gaz choisi.

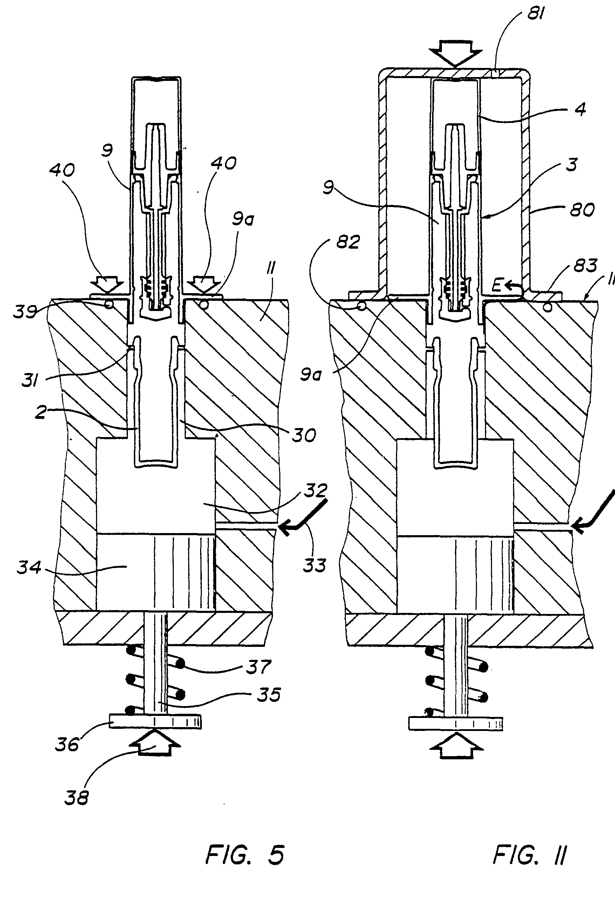

A cet effet, l'unité centrale de support et de transfert 11, illustrée par la fig. 5, également appelée table tournante ou linéaire, comporte des cavités 30 dans lesquelles sont logées les carpules 2 suspendues à des butés latérales 31. Chacune de ces cavités communique avec une chambre 32 alimentée en gaz choisi, représenté par la flèche 33, provenant d'une source de ce gaz sous pression. Cette chambre 32 contient un piston 34 agencé pour être actionné par une tige 35 munie d'une plaque d'appui 36 sur laquelle agit un ressort de poussée 37 et une force symbolisée par la flèche 38.

Lorsque le piston 34 est repoussé vers le haut, il coupe l'alimentation en gaz choisi et refoule le gaz de la chambre dans la cavité 30 en provoquant un flux de balayage autour des parois de la carpule. Lorsque l'injecteur est amené au-dessus de la carpule, on retrouve les configurations des fig. 4A, 4B et 4C. Des joints d'étanchéité 39 sont prévus autour de l'ouverture supérieure des cavités 30, pour assurer l'étanchéité entre la capsule 9 et la table 11, et pour permettre d'injecteur le gaz choisi en surpression dans la carpule. L'étanchéité est maintenue grâce à un appui exercé dans le sens des flèches 40 sur les ailettes 9a de la capsule 9. Le piston 34 a un double but: dans une première phase il engendre un flux de gas choisi autour de la carpule et dans une seconde phase il repousse la carpule pour provoquer l'enclipsage de l'injecteur et de la carpule.

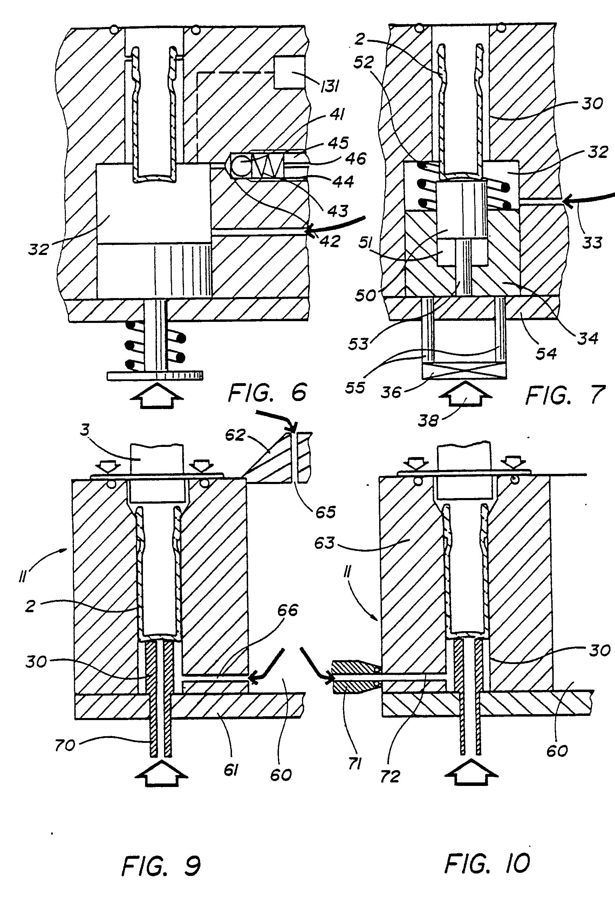

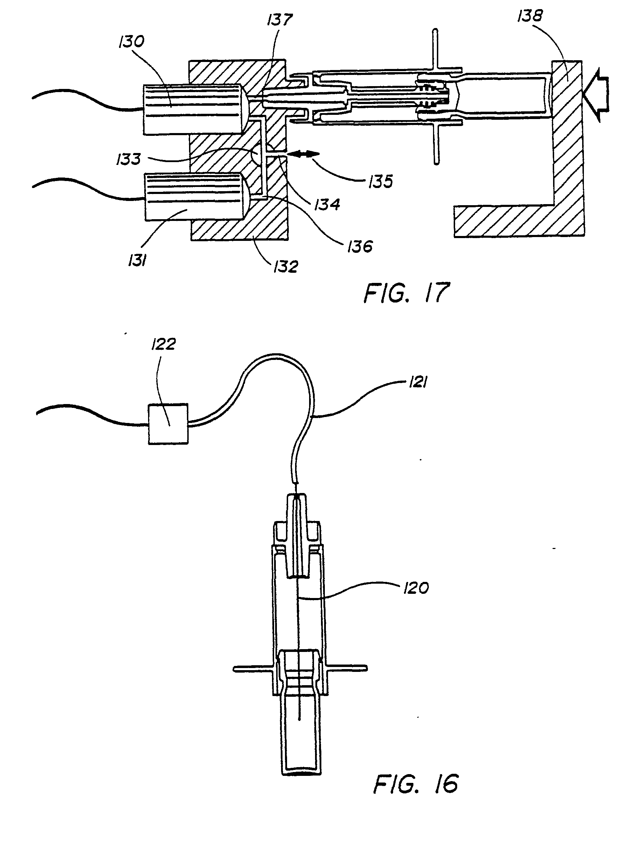

La cavité 30 ménagée dans la table 11 et illustrée par la fig. 6, diffère de la précédente en ce qu'elle comporte une soupape de surpression communiquant avec la chambre 32. Cette soupape comporte une bille 41 maintenue en appui contre un siège 42 par un ressort 43 bloqué dans un alésage 44 par une vis 45 traversée par un canal 46. La pression du ressort 43 réglable par la vis 45 permet de définir une pression maximale du gaz contenu dans la chambre 32, en fin de course du piston, et par conséquent la pression maximale du gaz contenu dans la carpule. Elle diffère également en ce qu'elle comporte un dispositif du type décrit en détail en référence à la fig. 17, pour permettre de détecter la pression partielle d'oxygène et/ou des particules solides.

La fig. 7 illustre une autre forme de réalisation de la cavité 30 contenant la carpule 2 et du mécanisme d'enclipsage de cette dernière sur l'injecteur. Dans cet exemple, la cavité ne comporte pas de butée de retenue de la carpule, qui repose sur un poussoir 50 logé à l'intérieur de la chambre 32.

Cette chambre contient comme précédemment un piston 34, qui, dans ce cas, comporte, une cavité 51 dans laquelle est partiellement logé le poussoir 50. Un ressort de compression 52 est disposé dans la chambre 32 en appui, d'une part contre la surface supérieure du piston 34, et d'autre part contre la surface supérieure de la chambre 32. Le poussoir 50 repose sur une tige verticale fixe 53 solidaire d'une plaque de base 54 ou en appui sur cette dernière. Cette plaque de base est de préférence traversée par deux tiges 55 munies comme précédemment d'une plaque d'appui 36 sur laquelle peut agir une force symbolisée par la flèche 38.

La carpule 2 se trouve initialement logée dans la cavité 30 en appui sur le poussoir 50. Lorsque la force 38 agissant sur la plaque d'appui 36 pousse le piston 34 vers le haut, l'alimentation en $CO_2$ représentée par la flèche 33 est tout d'abord coupée, puis le gaz contenu dans la chambre 32 est progressivement refoulé entre les parois extérieures de la carpule 2 et les parois intérieures de la cavité 30. Lorsque le piston 34 est soulevé suffisamment pour que le fond de la cavité 51 vienne en appui contre la base du poussoir 50, le piston 34 entraîne le poussoir 50 et exerce une poussée verticale ascendante sur la carpule 2, cette poussée étant suffisante pour provoquer l'enclipsage de la carpule et de l'injecteur (non représenté). Le ressort 52 joue le rôle de ressort de rappel destiné à refouler le piston 34 dans sa position initiale, le poussoir retournant d'office dans sa position de départ.

Dans l'exemple illustré par la fig. 8 l'unité centrale 11 de support et de transfert des carpules 2 et des injecteurs 3 comporte une cavité centrale 60 délimitée par une plaque de base 61, une plaque de couverture 62 et un bloc annulaire 63 dans lequel sont ménagées les cavités 30 destinées à recevoir les carpules 2. Mise à part l'existence d'une cavité annulaire 64 ménagée à la surface supérieure du piston 34, cavité servant à loger partiellement le ressort de compression 52, le mécanisme d'enclipsage  es carpules et des injecteurs, qui constitue si  ultanément le dispositif de refoulement du gaz choisi, est identique à celui décrit précédemment en référence à la fig 7.

Le gaz choisi est injecté dans la cavité centrale à travers un conduit 65 traversant la plaque de couverture 62. Un conduit 66 est ménagé à travers le bloc annulaire 63 pour faire communiquer la cavité 60 avec la chambre 32 localisée sous la cavité 30.

La fig. 9 illustre une variante de la réalisation selon la fig. 8 dans laquelle la chambre de dosage 32 du gaz utilisé pour provoquer un flux laminaire autour des parois de la carpule, a été supprimée. Dans cette réalisation le gaz choisi est introduit dans la cavité centrale 60 à travers le conduit 65 traversant la plaque de couverture 62. Le gazage des cavités 30 contenant les carpules 2 s'effectue d'une manière continue (et non plus de manière discontinue comme dans les réalisations précédentes) à travers le conduit d'amenée 66 qui fait communiquer la cavité 60 avec la cavité 30. Le mécanisme d'enclipsage des carpules et des injecteurs est constitué dans ce cas par un poussoir 70 traversant la plaque de base 61 de l'unité centrale de support et de transfert 11.

La fig. 10 illustre une autre forme de réalisation dans laquelle la cavité centrale 60 de stockage de gaz choisi a été supprimée et remplacée par des moyens d'alimentation extérieurs 71 raccordés à un conduit 72 qui communique avec la cavité 30 ménagée dans le bloc annulaire 63 de l'unité centrale 11.

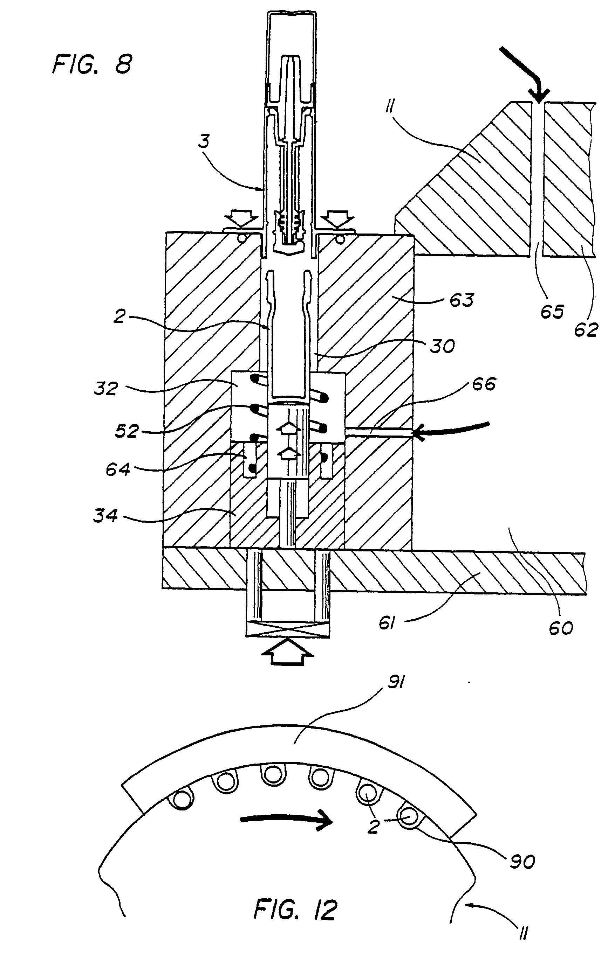

Une variante intéressante du dispositif tel qu'illustré par la fig. 3 est représentée par la fig. 11. Selon cette réalisation une cloche 80 est disposée par-dessus l'injecteur 3, pour maintenir ce dernier dans une enceinte contenant essentiellement du gaz choisi. Cette enceinte comporte avantageusement une ouverture 81 permettant la fuite du surplus de gaz contenu à l'intérieur de l'enceinte 80. Des joints d'étanchéité 82 sont ménagés sous le rebord de cette enceinte afin d'assurer une fermeture étanche entre ce rebord 83 et la surface supérieure de l'unité centrale 11. En revanche aucun joint d'étanchéité n'est prévu au niveau des ailettes 9a de la capsule 9, ce qui permet l'écoulement du gaz choisi contenu dans l'enceinte 30 vers l'intérieur de l'enceinte 80 dans le sens de la flèche E.

Cette enceinte 80 a été rapportée à titre d'exemple à un dispositif conforme à la réalisation selon la fig. 5. Il est toutefois évident qu'une telle enceinte pourrait être rapportée à n'importe quel autre dispositif décrit ci-dessus, étant donné que la présence de cette enceinte à pour but de créer une atmosphère de gaz choisi autour de l'injecteur pendant la phase d'enclipsage de la carpule et de cet injecteur.

D'une manière générale, le but du gazage des carpules, ainsi que de l'éventuel gazage des injecteurs est d'empêcher les particules ou l'oxygène de l'air de pénétrer dans l'atmosphère de gaz choisi surmontant le médicament liquide contenu dans la carpule. Pour éviter une telle pénétration, on crée dans tous les cas mentionnés précédemment un flux ascendant d'un gaz choisi, stérile, filtré et propre dans la cavité contenant la carpule, et on détermine la vitesse de ce flux ascendant de telle manière qu'elle soit supérieure à celle du flux laminaire descendant dans lequel se trouve l'ensemble de l'installation. La réglementation actuelle en matière de contrôle de médicaments à injecter impose un contrôle optique final destiné à déterminer l'éventuelle présence de particules dans le liquide médicamenteux. Le gazage tel que décrit en détail ci-dessus constitue un moyen efficace garantissant l'absence de particules solides dans le médicament, garantie qui, combinée à la présence d'un filtre que le médicament liquide est contraint de traverser avant d'être injecté, permet de supprimer en toute sécurité cette obligation de contrôle optique final. En outre, le dispositif tel que décrit peut comporter une sonde à particules intégrée à l'installation ainsi qu'une sonde de mesure de la pression partielle d'oxygène contenu dans l'atmosphère que l'on introduit dans la carpule, lorsque le remplissage du médicament doit être fait en milieu anaérobique, de telle manière que l'on dispose au moment du remplissage et du montage de la seringue de toutes les indications nécessaires pour rendre totalement superflu ce contrôle optique final particulièrement coûteux en temps et en matériel.

Pour amener les carpules dans les différentes stations de traitement, on peut prévoir divers dispositifs d'acheminement. La fig. 12 illustre un mode de réalisation dans lequel l'unité centrale 11 de support et de transfert des composants est constituée par une table rotative dont la surface périphérique est équipée d'une série d'encoches 90 conçues pour contenir les carpules 2, ces dernières étant retenues dans les encoches grâce à un guide fixe 91 qui obture ces encoches.

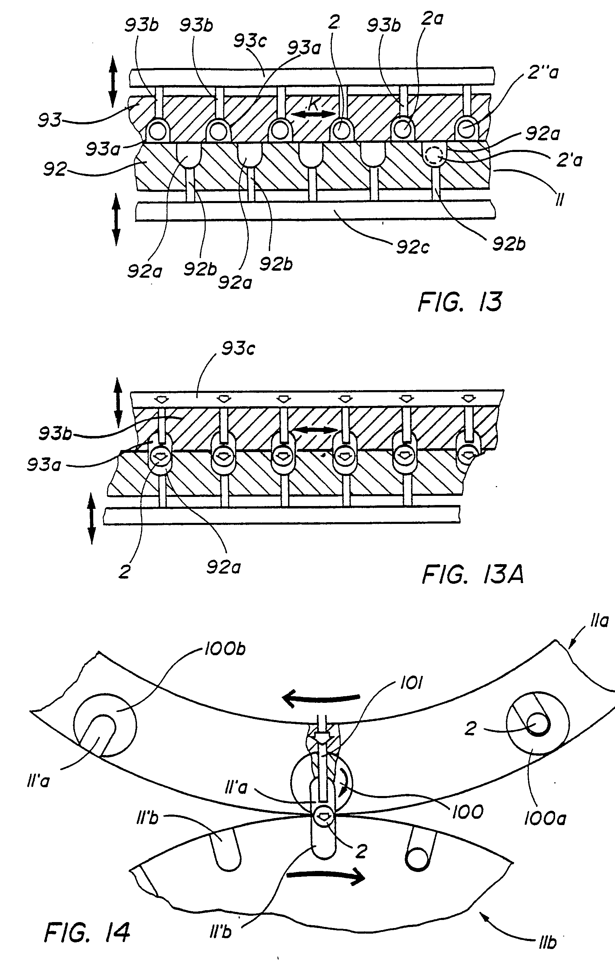

La fig. 13 illustre une autre forme de réalisation dans laquelle l'unité centrale de support et de transfert 11 est constituée par une table de transfert linéaire constituée d'un premier bloc 92 fixe et d'un second bloc 93 mobile dans le sens de la double flèche K. Les deux blocs 92 et 93 comportent respectivement une série d'encoches 92a et 93a dont chacune coopère avec un poussoir respectivement 92b et 93b, chaque série de poussoirs étant reliée par une réglette respectivement 92c et 93c. La coopération entre les différentes éléments permet de faire avancer les carpules 2 dans une direction parallèle au sens de déplacement du bloc 93 en les faisant passer alternativement d'une encoche 93a à une encoche 92a et réciproquement. En effet, une carpule 2a initialement logée dans une encoche 93a est amenée, lorsque le bloc 93 se déplace de la gauche vers la droite en face d'une encoche 92a du bloc 92. Le poussoir correspondant 93b pousse la carpule 2a dans la cavité 92a disposée en regard où elle occupera la position 2'a représentée en traits interrompus. Le bloc 93 effectuera un déplacement inverse de droite vers la gauche jusqu'au moment où l'encoche 93a suivante sera en face de l'encoche 92a contenant la carpule 2'a. Le poussoir correspondant 92b refoulera la carpule 2'a dans cette encoche où elle occupera la position 2''a. Ce déplacement alternatif permet par conséquent d'engendrer un déplacement linéaire ou rotatif des carpules, et leur acheminement dans les différents postes de traitement.

La fig. 13A représente une phase intermédiaire dans laquelle l'ensemble des poussoirs 93b actionné par la réglette correspondante 93c refoule l'ensemble des carpules 2 des encoches 93a vers les encoches 92a disposées en regard.

La fig. 14 illustre un moyen de transfert des carpules 2 d'une unité centrale de support et de transfert 11a vers une seconde unité de transport 11b. Pour permettre ce transfert chacun des deux éléments 11a et 11b comporte respectivement des encoches périphériques 11'a et 11'b. Etant donné que l'opération de gazage, pour qu'elle soit efficace, impose que les carpules soient momentanément logées dans une cavité cylindrique dont la profondeur est supérieure à la hauteur desdites carpules, les encoches 11a doivent être équipées de moyens permettant leur fermeture. Ces moyens peuvent être constitués par des portes rotatives 100 représentées en position fermée (référence 100a) et en position ouverte (référence 100b). Un poussoir 101 agit sur la carpule 2 pour la refouler de l'encoche 11'a dans l'encoche 11'b amenée en regard de l'encoche 11'a par le jeu des rotations inverses des deux éléments 11a et 11b.

Les portes rotatives 100 peuvent également être remplacées par tout autre dispositif de fermeture par exemple une porte à clapet ou à guillotine, un guide fixe extérieur etc.... L'ouverture des portes et la commande de l'éjecteur peuvent être réalisées par tout moyen mécanique connu en soi tel que came, rampe de guidage, dispositif électromagnétique etc....

La mise en place de l'injecteur sur la carpule, et plus particulièrement la phase qui débute au moment où le second point d'étanchéité est atteint (voir fig. 4C), engendre une réduction du volume intérieur réservé au gaz choisi surmontant le médicament liquide. Cette réduction de volume se traduit par une compression de ce gaz choisi. Si la réduction du volume due à l'insertion de l'injecteur n'est pas suffisante pour induire une surpression appréciable à l'intérieur de la carpule, on peut comme mentionné précédemment, accroître la surpression soit pendant, soit après le remplissage de la carpule, au cours d'une phase de mise sous pression.

Une telle surpression, qu'il est particulièrement facile d'obtenir et de conserver grâce à la conception originale de la seringue décrite, est mise à profit, comme cela sera décrit plus en détail ci-dessous, pour assurer le contrôle final des seringues après leur remplissage et la mise en place des injecteurs sur les carpules.

Indépendamment du problème du contrôle final, la surpression se justifie parce qu'elle permet d'obtenir et de maintenir la saturation du médicament liquide en gaz choisi, qui est habituellement l'anhydride carbonique. Lors du remplissage bien connu des ampoules de médicaments à injecter, on sature en général le médicament en gaz carbonique à la température ambiante voisine de 18 à 20°C. On obtient ainsi une saturation fixe connue. Sachant que le médicament se comporte approximativement comme l'eau et que l'eau absorbe sensiblement son propre volume de gaz carbonique à 15°C, pour accroître la saturation on peut refroidir le médicament au moment de son remplissage. Toutefois, cette solution n'est pas applicable à tous les médicaments dont certains ne se conservent pas à des températures inférieures à 15°C. L'autre solution pour augmenter la saturation consiste à accroître la pression. Dans la pratique, pour éviter qu'une surpression déterminée au moment de l'enclipsage de l'injecteur sur la carpule, le remplissage de cette dernière ayant été effectué à une température déterminée, soit totalement neutralisée au cours du stockage de la seringue, par suite d'un abaissement de la température ayant pour effet d'augmenter le taux de saturation, il convient de déterminer la surpression initiale de telle manière que la surpression résiduelle reste supérieure à zéro quelle que soit la température à laquelle le médicament est conservé.

Un autre but de la surpression est d'obtenir un écrasement de l'organe d'obturation qui se traduit par un serrage renforcé de cet organe contre les parois du col de la carpule. Ce serrage a pour conséquence d'accroître l'étanchéité de la carpule et de ce fait, d'augmenter la durée de conservation du médicament qu'elle contient, puisque les conditions de sursaturation en gaz choisi sont maintenues plus longtemps.

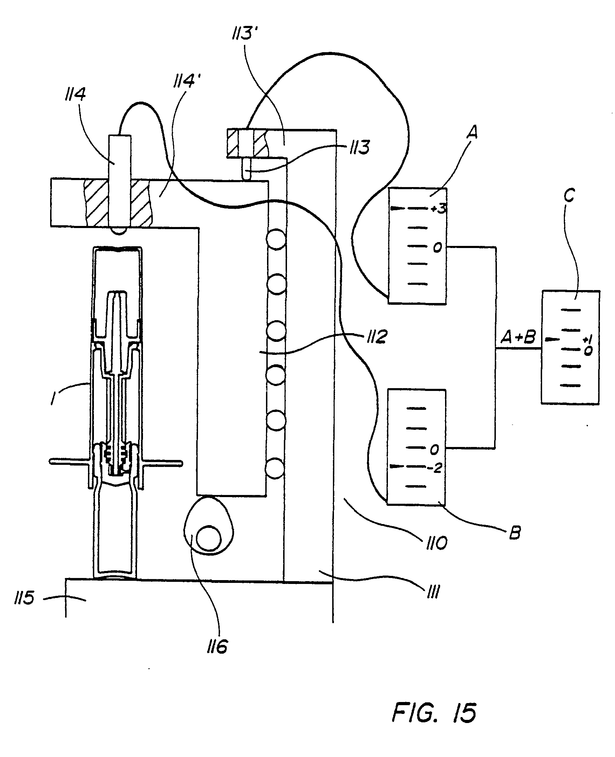

La station 19 de contrôle final des seringues ou station de contrôle dynamique est illustrée en détail par la fig. 15. Le dispositif 110 comprend un bâti vertical 111, une coulisse 112 mobile le long de ce bâti, et deux capteurs dimensionnels 113 et 114 portés respectivement par deux potences 113' et 114' solidaires respectivement du bâti et de la coulisse. Le bâti vertical est monté sur un support 115 agencé pour recevoir la seringue en vue dudit contrôle. Une came 116 commande les déplacements de la coulisse par rapport au bâti.

Le principe du contrôle est basé sur l'existence à l'intérieur de toute seringue conforme, d'une surpression dont on connaît la valeur. Une force exercée sur la carpule, -l'injecteur étant en appui sur le support 115, ou sur l'injecteur, la carpule étant en appui sur ce support, -provoque un enfoncement relatif de l'injecteur par rapport à la carpule. Grâce à la surpression régnant dans la carpule, la suppression de la force ayant provoqué cet enfoncement a pour conséquence un retour de l'injecteur dans sa position initiale.

Ce principe permet de contrôler toute une série de paramètres ayant trait aux composants de la seringue, leur assemblage et leur contenu. Grâce à ce contrôle dynamique, le fonctionnement à 100% des seringues est garanti, ce qui permet de supprimer en toute confiance le contrôle visuel des seringues tel qu'il est actuellement pratiqué sur les ampoules. Une sécurité complémentaire, citée pour mémoire, est obtenue par la présence d'un filtre dans chaque seringue, filtre qui permet à l'industrie pharmaceutique de garantir un produit conforme même après un stockage de très longue durée.

Le dispositif de contrôle opère de la manière suivante: la coulisse 112 descend jusqu'au moment où le capteur ou palpeur 114 entre en contact avec l'injecteur. A ce moment, le capteur 114 s'enfonce par rapport à la potence 114' de la coulisse. Le capteur 113 étant en appui contre la coulisse, sort progressivement au fur et à mesure que la coulisse descend par rapport à la potence 113'. Pendant la course du capteur 114, une unité de traitement de données, connectée à ce capteur, met à zéro la mémoire qui enregistre la différence des valeurs préalablement enregistrée par les deux capteurs dimensionnels. Lorsque le capteur 114 a complètement pénétré à l'intérieur de la potence 114', cette potence entre en contact avec l'injecteur. C'est à ce moment qu'une force F correspondant à une masselotte de masse M agit sur ce composant qui s'enfonce à l'intérieur de la carpule, le déplacement étant partiellement contrarié par la surpression (si la seringue est conforme). La différence des hauteurs indiquée par les deux capteurs est restée nulle tant que le capteur 114 s'est enfoncé dans la potence correspondante 114'. A partir du moment où la force F agit sur l'injecteur, le capteur 113 enregistre un déplacement de la coulisse. La valeur correspondante est affichée sur un indicateur A. Lorsque la came arrive à son point le plus bas, la masse M agit complètement sur l'injecteur. Lorsque la came commence à remonter, dans une première phase, le capteur 114 reste enfoncé dans la potence 114'. A la fin de la remontée de l'injecteur, le capteur 114 commence à sortir de la potence 114'. Cette fin de remontée de l'injecteur est mesurée par le capteur 114 et affichée sur un indicateur B.

Un indicateur C affiche la valeur de la somme algébrique des valeurs respectives affichées par les indicateurs A et B, ce qui permet de contrôler par lecture directe l'éventuelle différence entre la descente et la remontée de l'injecteur ayant subi une poussée axiale déterminée.

Le dispositif de contrôle dynamique décrit ci-dessus permet de contrôler:

a) la présence de chacun des composants de la seringue,

b) le volume de remplissage,

c) le défaut d'étanchéité par perte de gaz,

d) le siliconage des composants,

e) les tolérances de l'organe d'obturation,

f) les tolérances de la carpule,

g) l'enclipsage de l'injecteur,

h) le coulissement de l'injecteur par rapport à la carpule.

L'enfoncement insuffisant qui provoque un rejet automatique de la seringue, peut être dû aux raisons suivantes:

a) à l'absence d'un des composants,

b) au mauvais siliconage de la carpule,

c) à un serrage dû au diamètre intérieur de la carpule trop petit,

d) à un serrage dû au diamètre extérieur de la carpule trop grand,

e) au diamètre extérieur trop grand de l'organe d'obturation,

f) au diamètre intérieur trop petit de la capsule,

g) à un volume de remplissage trop important,

h) au mauvais coulissement de l'injecteur par rapport à la carpule.

L'enfoncement trop important de l'injecteur par rapport à la carpule peut être dû aux raisons suivantes:

a) au diamètre intérieur trop grand de la carpule,

b) au diamètre extérieur trop petit de l'organe d'obturation,

c) à un défaut de l'organe d'obturation,

d) à un défaut de la carpule,

e) à un volume de remplissage insuffisant,

f) à l'enclipsage incorrect de l'injecteur sur la carpule.

Si le retour de l'injecteur au-delà de sa position initiale est trop important, soit la carpule soit la capsule présente un défaut ou l'injecteur est mal enclipsé sur la carpule.

Si le retour de l'injecteur est inférieur à son enfoncement initial, l'étanchéité de la seringue est douteuse, son remplissage est incorrect ou des défauts de tolérance des composants provoquent un serrage de l'injecteur par rapport à la carpule.

On constate que ce dispositif de contrôle permet d'examiner pratiquement tous les paramètres de la seringue et de ses composants. La station de contrôle 19 est équipée de moyens permettant de refouler automatiquement toutes les seringues qui ne correspondant pas aux critères prédéterminés. Les seringues acceptées satisfont aux exigences les plus strictes, de sorte qu'elles sont prêtes à l'emploi et ne nécessitent plus aucun contrôle supplémentaire.

Le principe du dispositif de contrôle basé sur l'utlisation de capteurs dimensionnels et d'une masselotte de masse déterminée agissant sur l'injecteur, pourrait être remplacé par d'autres principes de mesure basés sur l'utilisation de capteurs de pression, de capteurs optiques ou similaires.

La fig. 16 illustre un dispositif permettant d'effectuer un contrôle statistique de la quantité de gaz contenu dans la carpule après assemblage des composants. La station correspondante est désignée par la référence 20 sur la fig. 3. Ce contrôle s'effectue de la manière suivante: on prélève de temps en temps une seringue 1. On introduit une aiguille 120 dans le canal axial de l'injecteur, et on perce l'organe d'obturation pour que l'extrémité libre de l'aiguille aboutisse dans l'espace disposé au-dessus du liquide et contenant le gaz choisi sous pression. Cette aiguille est raccordée par une tubulure souple 121 à un capteur volumétrique 122, ou plus simplement à une colonne d'eau, permettant de mesurer la quantité de gaz contenue dans la carpule.

La fig. 17 illustre un dispositif permettant d'effectuer un contrôle statistique de la qualité du gaz contenu dans la carpule. Ce dispositif comporte un compteur de particules 130 et un détecteur 131 à pression partielle de gaz. Le compteur de particules 130, du type optique ou à filtres, permet de contrôler l'éventuelle existence de particules solides dans le gaz choisi contenu dans la carpule. Le détecteur 131 est utilisé, dans ce cas, pour détecter l'éventuelle présence d'oxygène. Il peut être du type commercialisé sous la dénominations Orbisphere Modèle 2175 par Orbisphere Laboratories (Geneve—Suisse). Ce dispositif peut être monté dans la station 21 représentée schématiquement par la Fig. 3. Les deux détecteurs sont montés dans un bloc support 32 comportant une vanne 133, un canal de liaison 134 de cette vanne avec une alimentation en gaz choisi, utilisé pour purger l'installation, suggérée par la double flèche 135, un conduit de liaison 136 faisant communiquer cette vanne avec les deux détecteurs, et un conduit 137 de raccordement de la seringue 1 avec les capteurs. La vanne 133 permet de purger l'installation. Un poussoir 138 exerce une poussée sur la carpule pour permettre au gaz de s'échapper à travers les canaux de l'injecteur successivement vers les deux détecteurs. La seule méthode de détection de la présence d'oxygène utilisée actuellement est celle du bain KOH qu'il est impossible d'utiliser sur une ligne d'assemblage de seringues dans une station de contrôle automatique. L'utilisation de la méthode ci-dessus permet d'effectuer un contrôle efficace, immédiatement exploitable, de la qualité du gaz contenu dans les carpules.

La présente invention n'est pas limitée aux formes de réalisation décrites mais peut subir différentes modifications et se présenter sous diverses variants évidentes pour l'homme de l'art.

**Revendications**

1. Procédé de fabrication d'une seringue (1) préremplie à dose unitaire, comprenant d'une part une carpule (2) ouverte à une de ses extrémités, pourvue d'un col (5) de section rétrécie, et destinée à contenir un médicament à injecter et d'autre part un injecteur (3) composé d'une capsule (9) adaptable sur le col de la carpule et d'un piston-vanne (6) solidaire de la capsule et destiné à être inséré à l'intérieur de la carpule, procédé dans lequel on amène par deux voies différentes, dans une même station d'assemblage (16), la carpule contenant le médicament à injecter et l'injecteur, on positionne ces deux composants l'un au-dessus de l'autre et on les approche pour les assembler, et dans lequel on procède antérieurement à l'assemblage des composants, dans ladite station d'assemblage, à un gazage de la carpule (2) ou de la carpule (2) et de l'injecteur (3), caractérisé en ce qu'on effectue ce gazage en engendrant à l'intérieur d'une cavite (30) contenant entièrement ladite carpule, un courant de gaz circulant de bas en haut en balayant les parois extérieures de la carpule, et en provoquant à travers des canaux (7d, 8a) de l'injecteur un flux de gaz de balayage, en ce qu'on effectue un contrôle qualitatif et/ou quantitatif du gaz pendant ou après le gazage, et en ce qu'on effectue un contrôle final de la seringue (1) après l'assemblage des dite composants, en exerçant une poussée sur l'un de ces composants et en contrôlant au moins le retour de ce composant vers sa position initiale, ledit retour, étant déclenché par la suppression du gaz contenue dans la carpule.

2. Procédé selon la revendication 1, caractérisé en ce qu'au moins une phase de gazage de l'injecteur comprend le balayage des canaux par un flux ascendant de gaz surmontant initialement le liquide contenu dans la carpule, qui est refoulé vers les canaux lors de la mise en place de l'injecteur.

3. Procédé selon la revendication 2, caractérisé en ce que le courant de gaz balayant les parois extérieures de la carpule a une vitesse supérieure à la vitesse d'un flux laminaire dans lequel se trouve l'installation, et susceptible d'entraîner des particules contaminantes.

4. Procédé selon la revendication 2, caractérisé en ce qu'on provoque le courant de gaz de balayage en refoulant dans la cavité contenant la carpule, une quantité prédéterminée de gaz choisi stockée dans une chambre de dosage.

5. Procédé selon la revendication 2, caractérisé en ce qu'on provoque le courant de gaz de balayage en alimentant en continu la cavité contenant la carpule au moyen d'un gaz choisi.

6. Procédé selon l'une quelconque des revendications 4 ou 5, caractérisé en ce qu'on obture la cavité contenant la carpule et qu'on engendre ainsi le courant de gaz de balayage à une pression supérieure à la pression atmosphérique.

7. Procédé selon la revendication 2, caractérisé en ce que pour effectuer le gazage de l'injecteur on engendre, au cours d'au moins une phase de l'opération, un flux descendant de gaz de balayage, à travers les canaux de cet injecteur.

8. Procédé selon la revendication 7, caractérisé en ce qu'on maintient l'injecteur dans une chambre contenant du gaz choisi, pendant sa mise en place sur la carpule.

9. Procédé selon la revendication 1, caractérisé en ce qu'on effectue le contrôle de gaz sous forme statique par des interventions ponctuelles à fréquence déterminée.

10. Procédé selon la revendication 1, caractérisé en ce qu'on effectue le contrôle final des seringues en exerçant une force déterminée axialement sur la carpule ou sur l'injecteur préalablement assemblés, et en ce qu'on détecte à la fois la valeur de l'enforcement relatif de ces deux composants et la valeur du retour de ces composants dans leur position initiale.

11. Procédé selon la revendication 10, caractérisé en ce qu'on détecte également la somme algébrique des deux valeurs de l'enforcement relatif et du retour des composants dans leur position initiale.

12. Procédé selon la revendication 10, caractérisé en ce que la force déterminée est exercée par une masselotte de masse prédéterminée.

13. Dispositif pour la mise en oeuvre du procédé selon la revendication 1, comportant des moyens pour gazer la carpule (2) ou la carpule (2) et l'injecteur (3), avant et pendant l'assemblage de ces deux composants, caractérisé en ce que lesdits moyens sont agencés pour engendrer à l'intérieur d'une cavité (30) contenant entièrement ladite carpule (2), un courant de gaz circulant de bas en haut en balayant les parois extérieures de la carpule et pour provoquer à travers des canaux (7d, 8a) de l'injecteur (3) un flux de gaz de balayage, et en ce qu'il comprend également des moyens pour effectuer un contrôle qualitatif et/ou quantitatif du gaz pendant ou après le gazage, et des moyens pour effectuer un contrôle final de la seringue, après l'assemblage des composants, en exerçant une poussée sur l'un des composants et en contrôlant au moins le retour de ce composant vers sa position initiale.

14. Dispositif selon la revendication 13, caractérisé en ce que la cavité de gazage (30) a une profondeur supérieure à la hauteur de la carpule (2), et en ce qu'il comporte des moyens pour amener un gaz choisi dans cette cavité et des moyens pour engendrer un courant de balayage de ce gaz, circulant de bas en haut entre les parois de la cavité et celles de la carpule.

15. Dispositif selon la revendication 14, caractérisé en ce que la cavité de gazage (30) comporte une chambre de dosage (32) ménagée sous la cavité de gazage, cette chambre de dosage étant alimentée en gaz choisi en contenant initialement un volume prédéterminé de ce gaz à une pression donnée.

16. Dispositif selon la revendication 14, caractérisé en ce que la cavité de gazage est ménagée dans une unité centrale de support (11) et de transfert, et en ce que cette unité centrale se présente sous la forme d'une table circulaire rotative ou rectiligne à déplacement linéaire.

17. Dispositif selon la revendication 16, caractérisé en ce que l'unité centrale (11) de support et de transfert comporte plusieurs cavités (90), chacune de ces cavités étant constituée par une entaille ménagée à la périphérie de ladite unité et comportant des moyens pour fermer sélectivement cette entaille pour réaliser une cavité cylindrique.

18. Dispositif selon la revendication 17, caractérisé en ce que l'unité centrale (11) de support et de transfert se décompose en deux blocs adjacents (92, 93) dont l'un est fixe et dont l'autre est mobile en translation, chacun de ces blocs comportant une série d'encoches (92a, 93a) disposées de manière à pouvoir être amenées en regard les unes des autres afin que les carpules et/ou les injecteurs et/ou les seringues puissent être transférées d'une encoche à l'autre et déplacées linéairement avec le bloc mobile en translation.

19. Dispositif selon la revendication 17, caractérisé en ce que l'unité centrale (11a) de support et de transfert est associée à une seconde unité de transport (11b), et comporte une série d'encoches périphériques (11'a) comportant chacune une porte rotative (100) agencée pour coopérer avec l'encoche correspondant pour former ladite cavité de gazage (30).

20. Dispositif selon la revendication 13, caractérisé en ce que les moyens de contrôle final comportent deux capteurs dimensionnels (113, 114) montés l'un sur un bâti fixe (111), l'autre sur une coulisse mobile (112) axialement par rapport à ce bâti fixe.

21. Dispositif selon la revendication 20, caractérisé en ce que les moyens de contrôle final comportent trois indicateurs (A, B, C) agencés pour indiquer respectivement l'enfoncement relatif de la carpule (2) et de l'injecteur (3), le retour de l'injecteur (3) par rapport à la carpule (2) et la somme algébrique des deux valeurs précédentes.

22. Dispositif selon la revendication 13, caractérisé en ce que les moyens pour effectuer un contrôle quantitatif du gaz contenu dans la carpule comportent un capteur volumétrique (122) et des moyens pour mettre en communication l'intérieur de la carpule avec ce capteur.

23. Dispositif selon la revendication 13, caractérisé en ce que les moyens pour effectuer un contrôle qualitatif du gaz contenu dans la carpule comportent un compteur de particules (130) et/ou un détecteur (131) à pression partielle de gaz.

24. Dispositif selon la revendication 13, caractérisé en ce que la cavité de gazage (30) est raccordée à un détecteur (131) à pression partielle de gaz et/ou à un compteur de particules.

**Patentansprüche**

1. Verfahren zur Herstellung einer mit Einheitsdosis vorgefüllten Spritze (1), die einerseits eine an einem ihrer Enden offene Ampulle (2) aufweist, die mit einem Hals (5) mit eingeengtem Querschnitt versehen und dazu bestimmt ist, ein zu injizierendes Medikament aufzunehmen, und andererseits einen Injektor (3), bestehend aus einer an den Hals der Ampulle anpaßbaren Kapsel (9) und einem mit der Kapsel verbundenen Kolbenventil (6), das zum Einsetzen in die Ampulle vorgesehen ist, ein Verfahren, bei welchem man auf zwei verschiedenen Wegen an derselben Montagestation (16) die Ampulle, die das zu injizierende Medikament enthält und den Injektor heranführt, diese zwei Bestandteile übereinander zu liegen bringt und sie dann verschiebt, um sie zusammenzubauen; und bei welcher man vor dem Zusammenbau der Bestandteile in dieser Montagestation eine Begasung der Ampulle (2), oder der Ampulle (2) und des Injektors (3) durchführt, dadurch gekennzeichnet, daß man die Begasung durchführt, indem man im Inneren eines die Ampulle vollständig aufnehmenden Hohlraumes (30) eine Gasströmung bewirkt, die sich von unten nach oben bewegt, wobei sie die Außenwände der Ampulle überstreicht und indem man über die Kanäle (7d, 8a) des Injektors eine Gasströmung zum Spülen bewirkt, daß man eine qualitative und/oder quantitative Kontrolle des Gases während oder nach der Begasung durchführt, und daß man nach dem Zusammenbau der obigen Bestandteile eine Endkontrolle der Spritze (1) durchführt, indem man auf eines der Bestandteile einen Druck ausübt und indem man mindestens den Rückgang dieses Bestandteils in seine Ausgangslage überprüft, wobei der Rückgang durch den Überdruck des in der Ampulle enthaltenen Gases ausgelöst ist.

2. Verfahren nach Anspruch 1, dadurch gekennzeichnet, daß mindestens eine Phase der Begasung des Injektors die Spülung der Kanäle durch einen aufsteigenden Strom des Gases bewirkt, das die in der Ampulle vorhandene und beim Einsetzen des Injektors gegen die Kanäle gedrängte Flüssigkeit überlagert.

3. Verfahren nach Anspruch 2, dadurch gekennzeichnet, daß die Gasströmung, welche die Außenwände der Ampulle überstreicht, eine Geschwindigkeit hat, die höher ist als die Geschwindigkeit einer laminaren Strömung, welche die Installation umgibt, und die in der Lage ist, verunreinigende Partikel mitzureißen.

4. Verfahren nach Anspruch 2, dadurch gekennzeichnet, daß man die Gasströmung zum Spülen dadurch bewirkt, daß man in den die Ampulle aufnehmenden Hohlraum eine vorbestimmte, in einer Dosierkammer bereitgestellte Menge des ausgewählten Gases hineindrückt.

5. Verfahren nach Anspruch 2, dadurch gekennzeichnet, daß man die Gasströmung zum Spülen dadurch bewirkt, daß man kontinuierlich den die Ampulle enthaltenden Hohlraum mit einem ausgewählten Gas beaufschlagt.

6. Verfahren nach einem der Ansprüche 4 oder 5, dadurch gekennzeichnet, daß man den die Ampulle enthaltenden Hohlraum verschließt und so die Gasströmung zum Spülen unter einem Druck erzeugt, der höher als der atmosphärische Druck ist.

7. Verfahren nach Anspruch 2, dadurch gekennzeichnet, daß man, um die Begasung des Injektors zu bewirken, während mindestens einer Phase des Vorgangs eine absteigende Gasströmung zum Spülen durch die Kanäle dieses Injektors erzeugt.

8. Verfahren nach Anspruch 7, dadurch gekennzeichnet, daß man den Injektor während seines Einsetzens in die Ampulle in einer ausgewähltes Gas enthaltenden Kammer hält.

9. Verfahren nach Anspruch 1, dadurch gekennzeichnet, daß man die Überprüfung des Gases unter statischen Verhältnisses durchführt, indem man in einer bestimmten Häufigkeit zum richtigen Zeitpunkt kontrolliert.

10. Verfahren nach Anspruch 1, dadurch gekennzeichnet, daß man die Endkontrolle der Spritzen so durchführt, daß man axial eine bestimmte Kraft auf die Ampulle oder auf den Injektor ausübt, wobei man diese vorher zusammengebaut hat, und daß man gleichzeitig den Betrag der relativen Zusammendrückung dieser zwei Bestandteile und den Betrag des Rückgangs dieser Bestandteile in ihre Ausgangslage bestimmt.

11. Verfahren nach Anspruch 10, dadurch gekennzeichnet, daß man auch die algebraische Summe der zwei Beträge der relativen Eindringtriefe und des Rückgangs der Bestandteile in ihre Ausgangslange bestimmt.

12. Verfahren nach Anspruch 10, dadurch gekennzeichnet, daß man die bestimmte Kraft durch ein Fallgewicht mit vorbestimmter Masse aufbringt.

13. Vorrichtung zur Durchführung des Verfahrens nach Anspruch 1, welche Einrichtungen zum Begasen der Ampulle (2) oder der Ampulle (2), und des Injektors (3) vor und während des Zusammenbaues dieser zwei Bestandteile aufweist, dadurch gekennzeichnet, daß diese Einrichtungen dazu dienen, im Inneren eines die Ampulle (2) vollständig aufnehmenden Hohlraumes (30) eine Gasströmung zu bewirken, die sich von unten nach oben bewegt, wobei sie die Außenwände der Ampulle überstreicht und um über die Kanäle (7d, 8a) des Injektors (3) eine Gasströmung zum Spülen zu bewirken, und daß sie auch Einrichtungen zur Durchführung einer qualitativen und/oder quantitativen Kontrolle des Gases während oder nach der Begasung aufweist, und Einrichtungen, um nach dem Zusammenbau der Bestandteile eine Endkontrolle der Spritze durchzuführen, indem man auf eines der Bestandteile einen Druck ausübt und indem man mindestens den Rückgang diese Bestandteils in seine Ausgangslage überprüft.

14. Vorrichtung nach Anspruch 13, dadurch gekennzeichnet, daß der Hohlraum (30) für die Begasung eine Tiefe aufweist, die größer ist als die Höhe der Ampulle (2), und daß sie Einrichtungen für die Zuführung eines ausgewählten Gases in diesen Hohlraum aufweist, sowie Einrichtungen zur Erzeugung einer Spülströmung mit diesem Gas, welche sich zwischen den Wänden des Hohlraumes und denen der Ampulle von unten nach oben bewegt.

15. Vorrichtung nach Anspruch 14, dadurch gekennzeichnet, daß der Begasungshohlraum (30) einen unter dem Begasungshohlraum angeordneten Dosierraum (32) aufweist, wobei man diesen Dosierraum mit ausgewähltem Gas beschickt, und der jeweils ein vorbestimmtes Volumen dieses Gases unter einem gegebenen Druck enthält.

16. Vorrichtung nach Anspruch 14, dadurch gekennzeichnet, daß der Begasungshohlraum in einer zentralen Einheit (11) zur Halterung und zum Transport vorgesehen ist und daß diese zentrale Einheit die Form eines runden drehbaren oder eines geraden, linear verschiebbaren Tisches hat.

17. Vorrichtung nach Anspruch 16, dadurch gekennzeichnet, daß die zentrale Einheit (11) zur Halterung und zum Transport mehrere Hohlräume (90) aufweist, wobei jeder dieser Hohlräume eine an der Peripherie dieser Einheit angebrachte Aussparung bildet, und der Einrichtungen aufweist, um selektiv diese Aussparung zu verschließen, so daß ein zylindrischer Hohlraum ensteht.

18. Vorrichtung nach Anspruch 17, dadurch gekennzeichnet, daß sich die zentrale Einheit (11) zur Halterung und für den Transport in zwei benachbarte Einheiten (92, 93) aufteilt, wovon die eine fest ist und die andere in Form einer Parallelverschiebung beweglich ist, wobei jedes dieser Elemente eine Reihe von Aussparungen (92a, 93a) aufweist, die so angeordnet sind, daß man sie einander gegenüber stellen kann, um die Ampullen und/oder die Injektoren und/oder die Spritzen von einer Aussparung in die andere übergeben und mit dem parallel verschiebbaren Element linear weiterbewegen zu können.

19. Vorrichtung nach Anspruch 17, dadurch gekennzeichnet, daß die zentrale Einheit (11'a) zur Halterung und zum Transport mit einer zweiten Transporteinheit (11b) verbunden ist, und eine Reihe von peripheren Ausparungen (11a) aufweist, wobei jede mit einem drehbaren Verschluß (100) versehen ist, der so ausgebildet ist, daß er mit der entsprechenden Aussparung zusammenarbeitet, um den Begasungshohlraum (30) zu bilden.

20. Vorrichtung nach Anspruch 13, dadurch gekennzeichnet, daß die Einrichtungen zur Endkontrolle zwei dimensionsbezogene Sensoren (113, 114) aufweisen, wovon der eine an einem festen Gestell (111) und der andere an einer bezüglich dieses festen Gestells beweglichen Kulisse (112) montiert ist.

21. Vorrichtung nach Anspruch 20, dadurch gekennzeichnet, daß die Einrichtungen zur Endkontrolle drei Indikatoren (A, B, C) aufweisen, die dazu dienen, jeweils die relative Eindringtiefe der Ampulle (2) und des Injektors (3), den Rückgang des Injektors (3) bezüglich der Ampulle (2) und die algebraische Summe der zwei vorhergehenden Werte anzuzeigen.

22. Vorrichtung nach Anspruch 13, dadurch gekennzeichnet, daß die Einrichtungen zur Durchführung einer qualitativen Kontrolle des in der Ampulle enthaltenen Gases einen volumetrischen Sensor (122) und Hilfsmittel aufweisen, um das Innere der Ampulle mit dem Sensor in Verbindung zu bringen.

23. Vorrichtung nach Anspruch 13, dadurch gekennzeichnet, daß die Einrichtungen zur Durchführung einer qualitativen Kontrolle des in der Ampulle enthaltenen Gases einen Partikelzähler (130) und/oder eine Anzeige (131) für den Partialdruck des Gases aufweisen.

24. Vorrichtung nach Anspruch 13, dadurch gekennzeichnet, daß der Begasungshohlraum (30) mit einer Anzeige (131) für den Partialdruck des Gases und/oder einem Partikelzähler verbunden ist.

**Claims**

1. Method for manufacturing a prefilled single-dose syringe (1) comprising on one hand an ampoule (2) open at one end provided with a neck (5) of constricted section, for containing a medicine to be injected, and on the other hand an injector (3) composed of a capsule (9) adaptable to the neck of the ampoule and a piston-valve (6) solid with the capsule and adapted for insertion into the ampoule, method wherein the ampoule containing the medicine to be injected and the injector are conveyed via different paths to the same assembling station (16), these two components are positioned above one another and brought together for assembly, and wherein gassing of the ampoule (2) or of the ampoule (2) and of the injector (3) is effected prior to assembly of these components in said assembling station, characterized in that said gassing is effected by producing within a cavity (30) containing the entire ampoule a stream of gas circulating upwards from the bottom while sweeping the outer wall surfaces of the ampoule, and by creating a sweeping gas stream through the channels (7d, 8a) of the injector, in that a qualitative control and/or a quantitative control of the gas is effected during or after gassing, and in that a final control of the syringe (1) is effected after assembling said components, by exerting a thrust on one of these components and by controlling at least the return movement of this component towards its initial position, said return being released by the suppression of the gas contained in the ampoule.

2. Method according to Claim 1, characterized in that at least one phase for gassing the injector comprises said sweeping said channels with an ascending stream of a gas which initially rises above the liquid contained in the ampoule, which is expelled towards the channels when the injector is set in place.

3. Method according to Claim 2, characterized in that the gas stream sweeping the outer walls of the ampoule has a speed which is greater than the speed of a laminar flow in which the installation is placed, and which is capable of keeping away contaminating particles.

4. Method according to Claim 2, characterized in that the sweeping gas stream is produced by delivering into the cavity containing the ampoule a predetermined amount of a selected gas stored in a measuring chamber.

5. Method according to Claim 2, characterized in that the sweeping gas stream is produced by continuously supplying gas to the cavity containing the ampoule.

6. Method according to any one of Claims 4 or 5, characterized in that said cavity containing the ampoule is closed off and that the sweeping gas stream is thus produced at a pressure greater than atmospheric pressure.

7. Method according to Claim 2, characterized in that the injector is gassed by creating a descending sweeping gas stream through said channels of the injector, during at least one phase of the operation.

8. Method according to Claim 7, characterized in that the injector is maintained in a chamber containing a selected gas while it is set in place on the ampoule.

9. Method according to Claim 1, characterized in that the gas is controlled statistically by punctual interventions at a determined frequency.

10. Method according to Claim 1, characterized in that final control of the syringes is effected by exerting a given force axially on the ampoule or on the injector which have been previously assembled, and in that the value of the relative penetration of these two components and the value of the return movement of these components to their position are both determined.

11. Method according to Claim 10, characterized in that the algebraic sum of the two values of the relative penetration and the return movement of said components to their initial position are both determined.

12. Method according to Claim 10, characterized in that the given force is exerted with an inertia weight of predetermined mass.

13. Installation for carrying out the method according to Claim 1, comprising means for gassing the ampoule (2) and/or the ampoule (2) and the injector (3), before or after assembling these two components, characterized in that said means are arranged to produce within a cavity (30) containing the entire ampoule (2) a stream of gas circulating from the bottom upwards and sweeping the outer wall surfaces of the ampoule and to produce a sweeping gas stream through channels (7d, 8a) of the injector (3) in that it likewise comprises means for effecting a qualitative control and/or a quantitative control of the gas during or after gassing, and in that it further comprises means for effecting a final control of the syringe, after assembly of the components, by exerting a thrust upon one of the components and by controlling at least the return movement of this component towards its initial position.

14. Installation according to Claim 13, characterized in that the gassing cavity (30) has a depth greater than the height of said ampoule (2), and in that it comprises means for bringing a selected gas into said cavity and means for producing a sweeping stream of this gas, circulating from the bottom upwards between the walls of said cavity and those of the ampoule.

15. Installation according to Claim 14, characterized in that the gassing cavity (30) comprises a measuring chamber (32) arranged under the gassing cavity, supplied with a selected gas and initially containing a predetermined volume of this gas a given pressure.

16. Installation according to Claim 14, characterized in that the gassing cavity is arranged in a central support and transfer unit (11), and in that said central unit is in the form of a circular rotating table or a rectilinear, linearly displaced table.

17. Installation according to Claim 16, characterized in that the central support and transfer unit (11) comprises several cavities (90) each consisting of a recess provided at the periphery of said unit and comprising means for selectively closing off this recess to form a cylindrical cavity.

18. Installation according to Claim 17, characterized in that the central support and transfer unit (11) is divided into two adjacent blocks (92, 93) of which one is fixed and the other is mobile in translation, each of these blocks comprising a series of recesses (92a, 93a) disposed in such a manner that they may be brought to face one another so that the ampoules and/or the injectors and/or the syringes may be transferred from one recess to another and linearly displaced with the block which is mobile in translation.

19. Installation according to Claim 17, characterized in that the central support and transfer unit (11a) is associated with a second transport unit (11b), and comprises a series of peripheral recesses (11'a) each having a rotary door (100) adapted to cooperate with the corresponding recess to form said gassing cavity (30).

20. Installation according to Claim 13, characterized in that said final control means comprise two dimension sensor heads (113, 114) of which one is mounted on a fixed support frame (111), and the other on a slide (112) which is axially mobile with respect to this fixed support frame.

21. Installation according to Claim 20, characterized in that the final control means comprise three indicators (A, B, C) arranged to respectively indicate the relative penetration of the ampoule (2) and the injector (3), the return movement of the injector (3) with respect to the ampoule (2) and the algebraic sum of the two previous values.

22. Installation according to Claim 13, characterized in that the means for effecting quantative control of the gas contained in the ampoule comprise a volume gauge (122) and means for providing communication between the interior of the ampoule and this gauge.

23. Installation according to Claim 13, characterized in that the means for effecting control of the gas contained in the ampoule comprise a particle counter (130) and/or partial gas pressure detector (131).

24. Installation according to Claim 13, characterized in that the gassing cavity (30) is connected to a partial gas pressure detector (131) and/or a particle counter.

FIG. IA

FIG. IB

FIG. 2

FIG. 3

FIG. 4C

FIG. 4B

FIG. 4A

EP 0 185 027 B1

FIG. 5

FIG. II

5

FIG. 6

FIG. 7

FIG. 9

FIG. 10

FIG. 8

3

2

32

52

64

34

11

63

30

66

65

62

61

60

91

2

90

11

FIG. 12

FIG. 13

FIG. 13A

FIG. 14

FIG. 15

FIG. 17

FIG. 16